# EUROPÄISCHE PATENTSCHRIFT

(11) **EP 1 624 829 B1**
(45) Veröffentlichungstag und Bekanntmachung des Hinweises auf die Patenterteilung: **21.01.2009**
(21) Anmeldenummer: 03816998.3
(22) Anmeldetag: 20.05.2003
(51) Int. Cl.: A61F 2/16

(54) **FIXIERRING ZUR ENDO- ODER EXTRAKAPSULÄREN PROTHETISCHEN REKONSTRUKTION IM VORDERABSCHNITTSBEREICH DES MENSCHLICHEN AUGES**
FIXING RING FOR PERFORMING ENDOCAPSULAR OR EXTRACAPSULAR PROTHETIC RECONSTRUCTION IN THE FRONT SECTION AREA OF THE HUMAN EYE
BAGUE DE FIXATION UTILISEE EN RECONSTRUCTION PROTHETIQUE ENDO- OU EXTRACAPSULAIRE DANS LA ZONE DE LA SECTION AVANT DE L'OEIL HUMAIN

(43) Veröffentlichungstag der Anmeldung: 15.02.2006
(73) Patentinhaber: Hermeking, Heino, Dr., 42113 Wuppertal (DE)
(72) Erfinder: Hermeking, Heino, Dr., 42113 Wuppertal (DE)
(74) Vertreter: Meyer-Roedern, Giso
(86) Internationale Anmeldenummer: PCT/DE2003/001649
(87) Internationale Veröffentlichungsnummer: WO 2004/103221

(56) Entgegenhaltungen:
- EP-A- 0 319 154
- WO-A-01/89425
- WO-A-98/56314
- US-A- 4 657 546

## Beschreibung

Die Erfindung betrifft einen Fixierring zur endo- oder extrakapsulären prothetischen Rekonstruktion im Vorderabschnittsbereich des menschlichen Auges.

Intraoculare Prothesen erfüllen in erster Linie optische Aufgaben. Sie dienen in der Regel dem Ersatz der menschlichen Linse nach ihrer cataractchirurgischen Entfernung als dioptrisches Glied in der optischen Kette des Auges.

Für die Fixation einer intraocularen Prothese im Auge bieten sich folgende Möglichkeiten an (von anterior nach posterior):
1. kammerwinkelgestützt bzw. -fixiert; Vorderkammerlinse;
2. irisenklaviert, d. h. irisfixiert; Artisanlinse;
3. irisgetragen, d.h. durch die Pupille fixiert; Iriscliplinse;
4. sulcuspositioniert, d. h. bei Präsenz ausreichender Kapselreste und entsprechender Aufhängung (zonulae), Einlagerung der entsprechenden Linsenhaptiken in den Sulcus ciliaris;
5. endokapsuläre Positionierung, d. h. Einbringung der Intraocularlinse in den Linsenkapselsack;
6. Sklerafixation der Intraocularprothetik, d. h. transsklerale Einnähung und Verankerung der Linsenhaptik.

Bevorzugt wird im allgemeinen die endokapsuläre Positionierung, die allerdings einen weitgehend intakten Linsenkapselsack mit entsprechender Aufhängung voraussetzt. Bei fehlender Präsenz des Kapselsackes kommen andere Fixationstechniken zur alternativen Positionierung der Prothetik in Frage, wobei die einzelnen Positionierungen mit unterschiedlichen Risikofaktoren behaftet sind. Beispielsweise kann sich bei kammerwinkelgestützten Prothesen eine Hornhautdekompensation entwickeln.

Aus der WO 1998/056 314 A1 ist als Teil eines endokapsulären Prothethiksystems ein Fixierring für eine oder mehrere Blende(n) bekannt, mit der/denen eine künstliche Pupillenöffnung geschaffen wird. Der Fixierring weist einen flachen Ringkörper auf, der eine mittige kreisrunde Apertur hat und an dem zwei einander gegenüberliegende Haptikbügel ansetzen, die aus der Ringebene hoch und parallel dazu nach außen abgewinkelt sind. Die Haptikbügel dienen zur Verankerung an der/den Blende(n). Die zentrale Apertur des Fixierrings kann zur Anbringung einer optischen Prothetik genutzt werden.

Aus der WO 2001/089 425 A1 ist eine Halterung für eine Intraokularlinse bekannt, die die natürliche Linse ersetzt und bezogen auf deren vormalige Position nach hinten (a posteriori) versetzt implantiert wird. Die Halterung hat einen Ringkörper mit einer zentralen Apertur für die Aufnahme der künstlichen Linse und Haptikbügel zur Verankerung am Ciliarmuskel. Der Ringkörper ist über den Umfang geschlossen und durch Kerben geschwächt. Dadurch läßt sich die Halterung an ihrer Längsachse zusammenfalten und durch einen relativ kurzen Operationsschlitz implantieren. Die Haptikbügel sind offen.

Die individuellen Eigenschaften des Kapselsackes wie z. B. Äquatormaße haben zur Folge, daß man bei gegebenen Abmessungen der Blende(n), z. B. einem bestimmten Außendurchmesser, zum Zentrum des prothetischen Aufbaus eine Apertur realisiert, die von den individuellen, durch die anatomischen Vorgaben bestimmten Außendurchmessern geprägt ist. Ist beispielsweise der Äquator der Linsenkapsel kleiner als der Außendurchmesser der Prothetik, wird die Apertur verkleinert und entspricht nicht mehr der kreisförmigen Vorgabe. Aus diesem Grund ist es sinnvoll, als zentrales prothetisches Element den Fixierring einzubringen, der eine normierte Pupillengröße schafft. Grundsätzlich sollte dabei der Außendurchmesser des gesamten prothetischen Aufbaus nicht größer als der Kapselsackäquator sein.

In der Praxis hat es sich als schwierig erwiesen, den Fixierring in die prothetische Apertur der Blende(n) einzusetzen. Aufgabe der Erfindung ist es, einen Fixierring zu schaffen, der sich einfacher in anderweitige prothetische Teile einsetzen läßt.

Bei dem diese Aufgabe lösenden Fixierring ist der Ringkörper mittig geteilt, an der Teilung zusammenfaltbar und mit einander gegenüberliegenden seitlichen Verbindungsbügeln versehen, die elastisch sind, die Teilung überbrücken und den Ringkörper durch ihre Eigenelastizität in die flache Ursprungsstellung wieder aufzufalten geeignet sind.

Der Ringkörper des Fixierrings hat ein Formgedächtnis und eine elastische Formstabilität. Dadurch, daß er sich zusammenfalten läßt, ist ein kontrolliertes Einsetzen in eine je nach Außendurchmesser in den Abmessungen differierende innere, d. h. zentral gelegene Apertur eines prothetischen Außenaufbaus möglich. Der zusammenfaltbare Fixierring ist ein wichtiges Element, um die prothetische Konfiguration zu standardisieren und zu stabilisieren. Er federt dank der elastischen Verbindungsbügel in seine flache Ursprungsstellung zurück.

In die Apertur des Fixierrings kann eine Optik eingebracht, insbesondere eingesteckt oder eingeclipst werden. Der Fixierring kann auch in anderer Weise als Halterung für ein dioptrisches Element dienen, das durch den Fixierring wohlpositioniert ist.

Der erfindungsgemäße Fixierring kommt für endokapsuläre Prothetiksysteme zum Einsatz, die zum Aufbau einer Blende dienen, mit der es Irisabsenz oder Irisdefekte bzw. Irisdefizienten zu ersetzen gilt. Der Fixierring ist geeignet, an die Stelle des Fixierrings nach der WO 1998/056 314 A1 zu treten. Er bietet dabei dank seiner Zusammenfaltbarkeit entscheidende handhabungstechnische Vorteile.

Bei einer bevorzugten Ausführungsform ist der Ringkörper des Fixierrings im wesentlichen kreisringförmig und um die seitlich abstehenden Verbindungsbügel verbreitert.

Bei einer bevorzugten Ausführungsform nehmen die Verbindungsbügel etwa ein Achtel des Ringkörperumfangs ein. Die Verbindungsbügel haben eine schmale, abgerundet-rechteckige U-Form.

Bei einer bevorzugten Ausführungsvariante befinden sich die Haptikbügel zwischen den Verbindungsbügeln.

Bei einer bevorzugten Ausführungsform setzen die Haptikbügel beidends etwa in der radialen Mitte des Ringkörpers an. Ihre parallel zu der Ringebene sich erstreckende Partie ist gekrümmt. Sie reicht in der Mitte an den äußeren Rand des Ringkörpers heran, oder sie steht geringfügig nach außen darüber vor.

Bei einer bevorzugten Ausführungsform ist der Fixierring von zweizähliger Drehsymmetrie.

Bei einer bevorzugten Ausführungsform besteht der Fixierring aus gefärbtem Polymethylmethacrylat (PMMA) oder Polycarbonat. Trotz der Steifigkeit dieser Materialien läßt sich der Fixierring aufgrund seiner geometrischen Konfiguration zusammenfalten.

Die Erfindung wird im folgenden anhand von in der Zeichnung dargestellten Ausführungsbeispielen näher erläutert. Es zeigen:
- Fig. 1: die Draufsicht auf einen Fixierring;
- Fig. 2: eine Seitenansicht des Fixierrings mit Blick in Richtung II von Fig. 1; und
- Fig. 3: eine Seitenansicht des Fixierrings in einer alternativen Ausführungsform.

Der Fixierring besteht aus gefärbtem Polymethylmethacrylat (PMMA) oder Polycarbonat. Er hat einen flachen Ringkörper vom im wesentlichen der Form eines Kreisrings mit einem Außendurchmesser von 5 mm und mit einer mittigen, kreisrunden Apertur 10, die einen Durchmesser von 3 mm hat.

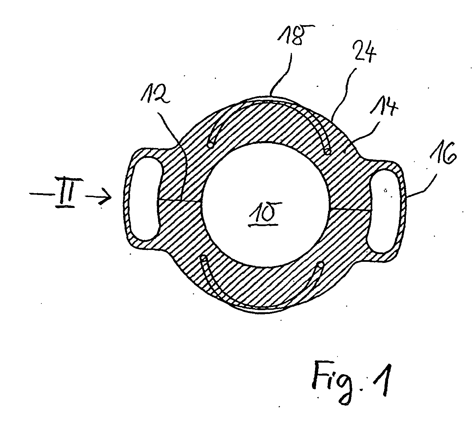

Der Ringkörper ist längs einer diagonalen geraden Trennlinie 12 mittig geteilt. Seine Halbteile 14 sind mit an den Ringkörper angeformten, seitlich davon abstehenden Verbindungsbügeln 16 verbunden, die die Trennlinie 12 überbrücken. Die Verbindungsbügel 16 liegen in der Ebene des Ringkörpers. Sie setzen spiegelsymmetrisch zu der Trennlinie 12 an den Halbteilen 14 des Ringkörpers an. Der Fixierring läßt sich an der Trennlinie 12 falten und dadurch in seinen äußeren Abmessungen verringern. Die Verbindungsbügel 16 sind so elastisch, daß der Fixierring in die flache Ursprungsstellung zurückzufedern tendiert, die in Fig. 1 dargestellt ist. In dieser Stellung stoßen die Halbringe 14 bündig aneinander, so daß sie sich zu einem ebenen Vollring ergänzen.

Zwischen den Verbindungsbügeln 16 setzen Haptikbügel 18 an den Halbringen 14 an. Die Haptikbügel 18 sind beidends aus der Ringebene senkrecht nach oben abgewinkelt. Ihre Bügelrücken 20 verlaufen auf gleicher Höhe parallel zu der Ringebene. Die Befestigungspunkte 22 der Haptikbügel 18 befinden sich etwa in radialer Mitte des jeweiligen Halbrings 14. Der Bügelrücken 20 ist nach außen gekrümmt. Er steht geringfügig über den äußeren Rand 24 des Ringkörpers nach außen vor.

Der Fixierring ist von zweizähliger Drehsymmetrie. Er geht bei einer 180°-Drehung um das Zentrum der Apertur 10 in sich selbst über.

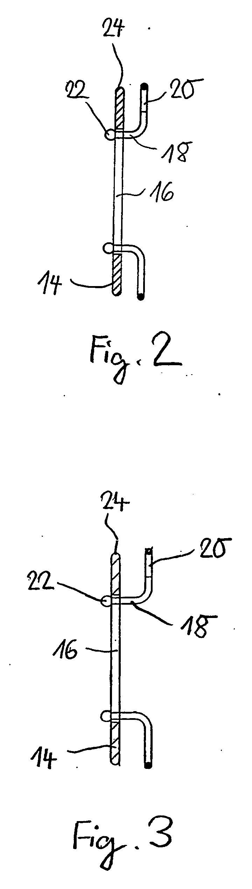

Verglichen mit dem Fixierring gemäß Fig. 1 und Fig. 2 sind bei dem Fixierring gemäß Fig. 3 die Haptikbügel 18 von der Ringebene weiter ausgestellt. Es paßt also ein Mehr an Prothetik zwischen den Ringkörper und die Haptikbügel des Fixierrings.

### Liste der Bezugszeichen

- 10: Apertur
- 12: Trennlinie
- 14: Halbring
- 16: Verbindungsbügel
- 18: Haptikbügel
- 20: Bügelrücken
- 22: Befestigungspunkt
- 24: Rand

## Patentansprüche

1. Fixierring zur endo- oder extrakapsulären prothetischen Rekonstruktion im Vorderabschnittsbereich des menschlichen Auges mit einem flachen Ringkörper, der eine mittige kreisrunde Apertur hat und an dem zwei einander gegenüberliegende Haptikbügel ansetzen, die aus der Ringebene hoch und parallel dazu nach außen abgewinkelt sind, **dadurch gekennzeichnet, daß** der Ringkörper mittig geteilt, an der Teilung zusammenfaltbar und mit einander gegenüberliegenden seitlichen Verbindungsbügeln (16) versehen ist, die elastisch sind, die Teilung überbrücken und den Ringkörper durch ihre Eigenelastizität in die flache Ursprungsstellung wieder aufzufalten geeignet sind.

2. Fixierring nach Anspruch 1, **dadurch gekennzeichnet, daß** der Ringkörper im wesentlichen kreisringförmig und um die seitlich abstehenden Verbindungsbügel (16) verbreitert ist.

3. Fixierring nach Anspruch 1 oder 2, **dadurch gekennzeichnet, daß** die Verbindungsbügel (16) etwa ein Achtel des Ringkörperumfangs einnehmen und eine schmale, abgerundet-rechteckige U-Form haben.

4. Fixierring nach einem der Ansprüche 1 bis 3, **dadurch gekennzeichnet, daß** sich die Haptikbügel (18) zwischen den Verbindungsbügeln (16) befinden.

5. Fixierring nach einem der Ansprüche 1 bis 4, **dadurch gekennzeichnet, daß** die Haptikbügel (18) beidends etwa in der radialen Mitte des Ringkörpers ansetzen, und daß ihre parallel zu der Ringebene sich erstreckende Partie (20) gekrümmt ist und in der Mitte an den äußeren Rand des Ringkörpers heranreicht oder geringfügig nach aussen darüber vorsteht.

6. Fixierring nach einem der Ansprüche 1 bis 5, **dadurch gekennzeichnet, daß** er von zweizähliger Drehsymmetrie ist.

7. Fixierring nach einem der Ansprüche 1 bis 6, **dadurch gekennzeichnet, daß** seine Apertur (10) einen Durchmesser von ca. 3 mm hat.

8. Fixierring nach einem der Ansprüche 1 bis 7, **dadurch gekennzeichnet, daß** er aus gefärbtem Polymethylmethacrylat (PMMA) oder Polycarbonat besteht.

## Claims

1. Fixation ring for endocapsulary or extracapsulary prosthetic reconstruction in the anterior section region of the human eye with a flat annular body that has a central circular aperture and on which two haptic stirrups that lie opposite one another are attached, which are angled upward from the ring plane, and outwards parallel to it, **characterized in that** the annular body is divided in the center, can be folded together at the division and is provided with lateral connecting stirrups (16) lying opposite one another, which are elastic, bridge the division and are suitable for unfolding the annular body into its flat original position again by means of their inherent elasticity.

2. Fixation ring according to claim 1, **characterized in that** the annular body is essentially circular and broadened by the connecting stirrups (16) that project away laterally.

3. Fixation ring according to claim 1 or 2, **characterized in that** the connecting stirrups (16) take up approximately one eighth of the circumference of the annular body, and have a narrow, rounded rectangular U shape.

4. Fixation ring according to one of claims 1 to 3, **characterized in that** the haptic stirrups (18) are situated between the connecting stirrups (16).

5. Fixation ring according to one of claims 1 to 4, **characterized in that** the haptic stirrups (18) are attached approximately in the radial center of the annular body, with both ends, and that their part (20) that extends parallel to the ring plane is curved and in the center, it reaches to the outer edge of the annular body, or it projects slightly outwards beyond it.

6. Fixation ring according to one of claims 1 to 5, **characterized in that** it has dual symmetry of rotation.

7. Fixation ring according to one of claims 1 to 6, **characterized in that** its aperture (10) has a diameter of approximately 3 mm.

8. Fixation ring according to one of claims 1 to 7, **characterized in that** it consists of dyed polymethyl methacrylate (PMMA) or polycarbonate.

## Revendications

1. Bague de fixation utilisée en reconstruction prothétique endo- ou extracapsulaire dans la zone du segment antérieur de l'oeil humain, avec un corps annulaire plat qui comporte une ouverture circulaire centrale et duquel partent deux anses haptiques opposées, s'élevant au-dessus du plan de la bague en étant pliées vers l'extérieur parallèlement à celui-ci, **caractérisée en ce que** le corps annulaire est centralement divisé, est repliable par la ligne de division et pourvu d'anses latérales de raccordement (16) opposées, lesquelles sont élastiques, enserrent la ligne de division et sont aptes à rabattre le corps annulaire dans sa position plane initiale par leur élasticité propre.

2. Bague de fixation selon la revendication 1, **caractérisée en ce que** le corps annulaire est sensiblement circulaire et élargi par les anses de raccordement (16) saillant latéralement.

3. Bague de fixation selon la revendication 1 ou la revendication 2, **caractérisée en ce que** les anses de raccordement (16) s'étendent sensiblement sur un huitième de la circonférence du corps annulaire et présentent une forme rectangulaire à angles arrondis en U étroit.

4. Bague de fixation selon l'une des revendications 1 à 3, **caractérisée en ce que** les anses haptiques (18) sont disposées entre les anses de raccordement (16).

5. Bague de fixation selon l'une des revendications 1 à 4, **caractérisée en ce que** les anses haptiques (18) partent sensiblement du centre radial du corps annulaire par leurs deux extrémités, et **en ce que** leur tronçon (20) s'étendant parallèlement au plan de la bague est cintré et correspond dans sa partie centrale au bord extérieur du corps annulaire ou dépasse légèrement de celui-ci vers l'extérieur.

6. Bague de fixation selon l'une des revendications 1 à 5, **caractérisée en ce qu'**elle présente une symétrie de rotation binaire.

7. Bague de fixation selon l'une des revendications 1 à 6, **caractérisée en ce que** son ouverture (10) a un diamètre de 3 mm environ.

8. Bague de fixation selon l'une des revendications 1 à 7, **caractérisée en ce qu'**elle est en polyméthylméthacrylate (PMMA) ou en polycarbonate coloré.
